# EUROPEAN PATENT APPLICATION

(11) **EP 2 380 514 A1**
(43) Date of publication of application: **26.10.2011**
(21) Application number: 10197469.9
(22) Date of filing: 31.12.2010
(51) Int. Cl.: A61B 17/86, A61B 17/72

(54) **Multiple canulated and self guided intramedullary nail**

(30) Priority: 20.04.2010 TR 201003130
(71) Applicant: Havitcioglu, Hasan, Izmir (TR)
(72) Inventor: Havitcioglu, Hasan, Izmir (TR); Savas, Düzel, Izmir (TR); Baran, Önder, Izmir (TR)
(74) Representative: Iskender, Ibrahim

(57) **Abstract**

The invention serves the purpose of detection devices, along with fractures of bones, fracture detection, limb lengthening, deformity, hotfixes, multiple grooves and channels in the structure with the help of a self-guided, the intramedullary nail from the outside without the need for systems to be added to the guide are available, facilitating the detection of intramedullary nail within the bone needed to determine x-ray (radiation) to reduce the amount of multi-channel use, and feature self-guided intramedullary nail found;

## Description

The invention is used in the treatment of orthopedic medical ailments, and extensions related to the detection of bone with surgical intervention

The invention in particular, serves the purpose of fixation devices, along with fractures of bones, fracture fixation, limb lengthening, deformity, correcting an available multi-channel, self-guided intramedullary nail associated with detection.

### Background of the Invention

Bone intramedullary nail is developed in order to use the fixation of fracture, limb lengthening, deformity correcting

The current technique, especially in the human body fracture or broken bone fragments for the fixation and treatment of other reasons, often surgical operation methods of fixation are available inside and outside. The fixation methods are used to perform the various materials.

Nowadays, many currently available model of intramedullary nails are used when applicable, the broken bone fragments are frequently in fixation of the fragman of broken bone needs to be treated using surgical methods. Many currently available model of intramedullary nails.

In the human body, the femur, tibia, humerus, fibula fractures of long bones, such as for surgical repair and stabilization of bone, a piece of the inner cavity of a suitable length of metal tubular intramedullary nail placement, known for many years in medical practice.

Surgical treatment is widely used as an application to determinate of bone fracture with interlocking intramedullary nail which is placed into the medulla of bone at the proximal and distal to the nail holes without the eye can see from the outside trying to fixation with the stability of screws.

Fixing screws cross at the nail for stabilitation of the intramedullary nail, holes pierced through the bone cortex and medulla.

Intramedullary nails are used today are usually close to the proximal end of a pair of transverse (side-width), hole (proximal holes) near the distal end and a pair of transverse (side-width), hole (distal hole) has.

Increase the speed of healing of bone fractures and bone tissue formation in the optimum conditions required to ensure the realization of the most appropriate manner.

One of the most important of these conditions is the union can be detected with intramedullary nail fixation of bone fracture in a rigid structure. Therefore, nail, bone-compatible format should be fixed. To fixation of the nail with the proper tack, and extending transversely through the bone, surrounding tissues is provided with transverse holes in the screw or bolt.

However, the process of intramedullary nail fixation for the screws; transverse screw holes on bone and nail holes with the precise measurement, the drilling of the most consistent, precise nail holes on the transverse localization (localization) and consistent with the purpose of determining the bone screws in the nail holes and from there to drop the nail must be placed on the transverse holes.

Nowadays, surgical treatment is an important problem encountered with the determination of broken bones, the bone screw holes in the intramedullary nail and the nail exactly on the transverse parts of the study of indetermination bone hole. The most critical part of the bone drilling process, the nail holes in the location of the transverse parts of the without eye can see, the center of the hole and the axis of the bone drilling section is determined.

Intramedullary nails in order to solve the mentioned problem on the transverse screw holes are easily identified by finding the most precise measurement complete bone and intramedullary nail for the right axes to cracking the structure of the various guidelines have been developed to help.

These guidelines determined by the screw holes with a nail in the transverse section of three planes full and in accordance with the distances and positions to determine. Technique used in the guides is associated with the proximal end of the intramedullary nail in the transverse (side-width) with the screw holes are detected.

Intramedullary nail is connected to the grid at the end of the proximal transverse screw holes in the identified locations, and positions of the proximal nail end distance to be found more easily. However, the distal part of the intramedullary nail holes are detected in the transverse screw locations are determined with difficulty Intramedullary nailing of the distal part of the determination of the positions of the screw holes are detected, the nail during insertion into the bone to the return or bend slightly depending on it can be difficult.

The majority of techniques aimed at solving the problems mentioned above, the transverse part of the intramedullary nail, the distal and proximal position of the screw holes are detected and the direction of the bone screws by drilling for fixation carried out to ensure the process is determined by using x-rays. For this purpose, the direction of x-rays to be controlled for the C-arm fluoroscopy is often used.

The most common technique used in the C-arm "free-hand" technique. This technique is mainly based on the surgeon's experience and steady hands In addition, the surgeon with this technique, with his hands while working in close proximity to a lot of x ray radiation (x-ray) exposure to lead.

U.S. patent No. 6,053,918 can be accepted as the state of the art "Apparatus and method for fastening an intramedullary nail to a bone" with the title of invention patents and one intramedullary nail on the inside (cavity) is controlled, aligned with the nail holes on the pre-built security, preferably biological Polyglycolic acid is a biodegradable material produced as a guide intramedullary nail fixation apparatus and method associated with this unit Intramedullary nail, screw and the fracture was detected in the bone, a broken bone in the anatomic structure of bone tissue formation in the realization of the most appropriate way to allow the fixing of a very precise measurement is required.

U.S. patent number 6,053,918 mentioned invention the guide apparatus 200 number, 218 numbers and 219 numbers will keep the rod through the intramedullary nail is placed in the inner part. During this process, the number 3 from the intramedullary nail 200 of the guide apparatus will occur as a result of contact with each other due to friction forces in the nail bone is likely to give a small amount of rotation or tilt motion In this case, nails, bone is located in an ideal position to be act as the alter.

The insertion and extraction process, loss during the operation time, cause to precise fixed position of the bone with fixation. No. 22 with the tip of the drill guide apparatus during drilling in order to create the guide channel is likely faulty drilling.

This method applied to the guide system, is obliged to be constituted by a row of nail holes compulsory In addition, the guide apparatus is used as material, bioabsorbable, preferably Polyglycolic acid material; bone tissue in the risk of infection is the cause of foreign body reactions.

U.S. patent No. 4,943,291 can be accepted as the state of the art, "Drilling feeler, in particular for positioning and securing a medullary nail" with the title of invention patents; medullary nail, and in particular to protect the position of the antenna is related to a drill. Inserted into the guide pin into the bone to create the form of an application with the invention includes several steps, during surgery when the surgeon will take a lot of time. In addition, more than one unit to create a guide to the use of more sensitive, requires experience and time.

U.S. patent No. 4,781,181 can be accepted as the state of the art, "Boring sensor for intramedullary nail intramedullary nail and Corresponding" with the title of invention patent, an intramedullary nail intramedullary nail for the drilling and related sensor.

Invention, the nail guide system is realized as a multiple of the nail apparatus created and controlled Nail apparatus formed additional guidance system, the manual control is provided with the help of this apparatus. Therefore, fixed with intramedullary nail after the bone in the best position to act is likely to take a position a little bit different

Although technically easier to place the intramedullary nail intramedullary nail in the proximal and distal bone screws are determined to find the holes go through the often is difficult. X-rays to determine bone in the nail holes on the use of large amounts should be very repetitive. Considering the amount of radiation people are exposed to X-rays taken from the body, both the patient and the surgical team's health is affected adversely. This is usually done with fluoroscopic guidance with intramedullary procedures for the patient and the surgical team at a disadvantage and the risk is very important.

During the reduction and intramedullary nail in transverse section of a broken bone in particular, through the distal screw holes and screws, bone screws a lot of time is spent. Especially in the distal screw holes found on the nail and the bone breaks, in accordance with spending a very prolonged duration of the operation. Therefore, patients are exposed to anesthesia for a longer period of time, increasing the risks of anesthesia. Prolongation of the duration of the surgery, the surgeon could be effect negatively the number of the day surgery, the number of examinations in the day of patients.

### Objectives of the Invention

The aim of the invention of the technique based on the current status of the structure of an intramedullary nail, the nail is controlled via the multiple (multiple) cannula system formed, to facilitate the determination of the nail in the bone, so the patient and the health care team to prevent further exposure to X rays and radiation.

The aim of the invention based on the current status of the technique using intramedullary nails to treat at least keep people as well as a greater number of physician examining a patien or to create opportunities with shortening the duration of surgery and anesthesia in patients exposed to..

The aim of the invention of the technique based on the current position, the fixation of fracture, limb lengthening, for correcting the deformity of the bone screw of intramedullary improvements identified through elimination of existing disadvantages.

Another purpose of the invention of surgical intervention was completed in less time with a more practical application to minimize the risk of infection from patients.

Another purpose of the invention are fixed in a broken bone intramedullary nail, with fixed by bone screws that may occur in the movement to eliminate the risk.

Another purpose of the invention using intramedullary nail fixation screws in the process of bone patients, the patient will consist of a minimum deformation of way, with the most precise measurements to create optimum conditions for stabilization whether the patient's healing.

Invention to achieve the aforementioned objectives, helps to detect fractures in bones with the devices, the fixation of fracture, limb lengthening, deformity, multiple grooves and channels in the structure with the help of a self-guided, the guide will be added outside the intramedullary nail can be used without the need for systems, multi-channel, self-guided intramedullary nail found.

Proximal end of the guide wire is applied to determine the appropriate point on the bone proximal to the guide for drilling by the way, the proximal end of the intramedullary nail 1 groove in, 1 groove, 1 groove angled surface, 1 Chute-out, 1 Fixing screw-in, 1 Fixing screw out;
proximal end of the guide wire is applied to determine the appropriate point on the bone proximal to the guide for drilling by the way, the proximal end of the intramedullary nail 2 groove in, 2 groove, 2 groove angled surface, 2 Chute-out, 2 Fixing screw-in, 2 Fixing screw out;
proximal end of the guide wire is applied to determine the appropriate point on the distal bone by drilling needed to guide the way, the proximal end of the intramedullary nail 1 Channel entrance, along the nail body 1 Channel, intramedullary nail distal end of the 3 groove, 1 Channel angled surface, 3 Fixing screw-in, 3 Fixing screw out;
proximal end of the guide wire is applied to determine the appropriate point on the distal bone is needed to guide the way by drilling, the proximal end of the intramedullary nail 2 Channel entrance, along the nail body 2 Channel, intramedullary nail distal end of the 4 groove 2 Channel angled surface, 4 Fixing screw-in, 4 Includes retaining screw out.

Invention, a preferred realization of the purposes mentioned in the broken bone are placed in the intramedullary nail to be inserted through the bone cavity to determine the path will guide the nail guide wire nail, the nail guide out of the distal end, the body of the nail along the nail guide channel, located in the proximal nail end include guidance with input provided.

Invention, in a preferred realization of the purposes mentioned, the shape and form of an intramedullary nail, place the bone to the anatomic structure of the suitability of the nail in the neck, the neck bone, depending on the angle to include various sizes of nails provided.

Invention, in a preferred realization of the purposes mentioned, the guide wire is detected, detecting and securing the cannulated intramedullary nail found in the bone screw, nail, without an additional guide tracks integrated, self-controlled manner with guidance that is provided

Invention, a preferred realization of the purposes mentioned in the nail grooves contained, angled surfaces of grooves, channels, inputs, and screw screw outputs, determined the appropriate angle to head out of the guide wire nails, nail outside the bone to ensure proper angle delmesini angle, shape and form contains are provided with.

Invention, a preferred realization of the purposes mentioned in the guide wire is detected, detecting and securing the cannulated intramedullary nail found in the bone screw, nail, without an additional guide tracks integrated, self-controlled manner with guidance that is provided.

Invention, a preferred realization of the purposes mentioned in the nail grooves contained, angled surfaces of grooves, channels, inputs, and screw screw outputs, determined the appropriate angle to head out of the guide wire nails, nail outside the bone to ensure proper angle delmesini angle, shape and form contains are provided with.

Invention, a preferred realization of the purposes mentioned in the guide wire is detected; the bone is provided with the desired angle and diameter drilling.

Invention, a preferred realization of the purposes mentioned in the guide wire is detected identified cannulated screw is passed through the screw into the entry, exit after leaving the bone screw, the output in the direction of the pass against the cortex is provided by the piercing.

### Description of the Drawings

Figure- 1; Persfective view of representation application of multiple cannulated and self guided intramedullary nail.
Figure- 2; In the representation application of the invention, cross-sectional view of the intramedullary nail where the chanells on the structure can be seen.
Figure- 3; View of the invention of the proximal intramedullary nail (top-head) end of the channel entrance holes from the top.
Figure-4; Cross-sectional and perspective view of the invention of the intramedullary nail which is inserted and fixed in to a broken long bone medulla.

### Reference Numbers:

- 1.: Proxi mal end
- 2.: Distal end
- 3.: Neck of the nail
- 4.: 1. Groove input
- 5.: 2. Groove input
- 6.: 1. Channel input
- 7.: 2. Channel input
- 8.: 1. Groove output
- 9.: 2. Groove output
- 10.: 1. Channel output
- 11.: 2. Channel output
- 12.: 1. Fixation screw input
- 13.: 2. Fixation screw input
- 14.: 3. Fixation screw input
- 15.: 4. Fixation screw input
- 16.: 1. Fixation screw output
- 17.: 2. Fixation screw output
- 18.: 3. Fixation screw output
- 19.: 4. Fixation screw output
- 20.: 1. Groove angled surface
- 21.: 2. Groove angled surface
- 22.: 1 Channel angled surface
- 23.: 2. Channel angled surface
- 24.: Nail guide input
- 25.: Nail body
- 26.: Nail neck angle
- 27.: Nail guide channel
- 28.: 1. Groove
- 29.: 2. Groove
- 30.: 1. Channel
- 31.: 2. Channel
- 32.: Nail guide output
- 33.: Nail guide wire
- 34.: Cannulated fixation screw
- 35.: 3. Groove
- 36.: 4. Groove
- 37.: Fixation guide wire
- 38.: Broken bone cavity

### Detailed Description of the Invention

The most accurate way to understand the working principle of the invention, the evaluation of all Figures (Figure-1, Figure-2, Figure-3, Figure-4) will be appropriate.

The application of the invention given in the figures is a system allows, finding exact localization of the holes on the intramedullary nail structure from outside easily, inserting the fixation screws in to the nail holes by drilling the bone and minumum x - ray exposure.

The general view of the invention of intramedullary nail is illustrated in Figure-1.

In Figure-1, the neck (3) between the proximal end of the nail (1) and the distal end (2) and the nail body (25) is located. The transverse diameter of the proximal end of the nail (1) is larger than the transverse diameter of the distal end (2) in order to achieve the ideal placement capability of the nail into the bone. The distal end (2) has a little conical geometry to ensure easier entry of the nail into the bone. On the proximal end (1) of the nail, there are first groove input (4) and second groove input (5). On the proximal end (1) of the nail, continuation of the first Groove input (4), there is first Groove (28), continuation of the second groove input (5), there is secoond groove (29). The first groove (28) is combined with the first fixation screw output (16) on the first groove angled surface (20). In the same way, the second groove (29) is combined with the first fixation screw output (17) on the first groove angled surface (21). Following the placement of the intramedullary nail into the bone, the fixation screws that are attached on the proximal end (1) will fix the nail to the bone by going out of the first fixation screw output (16) and second fixation screw output (17).

Nail guide wire (33) (Figure-1), is a wire which is sent to the bone medulla as a guide before the placement of the nail in to the bone. The intramedullary nail is inserted in to the broken bone by carrying forward on the nail guide wire (33) while the nail guide wire (33) is in the nail guide channel (27) (Figure-2). The nail guide wire (33) is guiding in the nail guide channel (27) between the nail guide output (32) and nail guide input (24).

The part where the proximal end (1) of the intramedullary nail and the nail body (25) combined with is nail neck (3) (Figure-1). The nail neck (3) has an optimum sized nail neck angle (26) for the purpose of positioning the intramedullary nail into the broken bone in the most appropriate way with the bone anatomic structure. (Figure-1)

In Figure-2, there is lengthwise cross-sectioned intramedullary nail where the channels in it can be seen.

In order to identify the gaps of fixation screw on the structure of the intramedullary nail inserted in to the broken bone, the fixation guide wire (37) enters in to first groove input (4), passes through the first groove (28) and can be oriented to the first groove output (8) owing to first groove angled surface (20). So, the bone can be drilled at first groove output (8) point by fixation guide wire (37).

The point of first groove output (8) where the fixation guide wire (37) drills the bone, provides the cannulated fixation screw (34) to get in to the bone and the intramedullary nail through the fixation guide wire (37) as first fixation screw input (12). The canullated fixation screw (34) that reaches to the intramedullary nail from the first fixation screw input (12), will be fixing the nail on the broken bone by following the way of first fixation screw output (16), getting out of the nail and drilling the bone.

In order to identify the gaps of fixation screw on the structure of the intramedullary nail inserted in to the broken bone, the fixation guide wire (37) enters in to second groove input (5), passes through the second groove (29) and can be oriented to the second groove output (9) owing to second groove angled surface (21). So, the bone can be drilled at second groove output (9) point by fixation guide wire (37).

The point of second groove output (9) where the fixation guide wire (37) drills the bone, provides the cannulated fixation screw (34) to get in to the bone and the intramedullary nail through the fixation guide wire (37) as second fixation screw input (13). The canullated fixation screw (34) that reaches to the intramedullary nail from the second fixation screw input (13), will be fixing the nail on the broken bone by following the way of second fixation screw output (17), getting out of the nail and drilling the bone.

In order to identify the gaps of fixation screw on the structure of the intramedullary nail inserted in to the broken bone, the fixation guide wire (37) enters in to first channel input (6), passes through the first channel (30) , reaches to the third groove (35) and can be oriented to the first channel output (10) owing to first channel angled surface (22). So, the bone can be drilled at first channel output (10) point by fixation guide wire (37).

The point of first channel output (10) where the fixation guide wire (37) drills the bone, provides the cannulated fixation screw (34) to get in to the bone and the intramedullary nail through the fixation guide wire (37) as third fixation screw input (14). The canullated fixation screw (34) that reaches to the intramedullary nail from the third fixation screw input (14), will be fixing the nail on the broken bone by following the way of third fixation screw output (18), getting out of the nail and drilling the bone.

In order to identify the gaps of fixation screw on the structure of the intramedullary nail inserted in to the broken bone, the fixation guide wire (37) enters in to second channel input (7), passes through the second channel (31), reaches to the fourth groove (36) and can be oriented to the second channel output (11) owing to second channel angled surface (23). So, the bone can be drilled at second t channel output (11) point by fixation guide wire (37).

The point of second channel output (11) where the fixation guide wire (37) drills the bone, provides the cannulated fixation screw (34) to get in to the bone and the intramedullary nail through the fixation guide wire (37) as fourth fixation screw input (15). The canullated fixation screw (34) that reaches to the intramedullary nail from the fourth fixation screw input (15), will be fixing the nail on the broken bone by following the way of fourth fixation screw output (19), getting out of the nail and drilling the bone.

In Figure-3, when looking at the top of proximal end (1) of the intramedullarry nail, guide ways of the fixation guide wire (37), fisrt groove input (4), second groove input (5) , first channel input (6), second channel input (7) and nail guide input (24) which is the guide way of nail guide wire(33) can bee seen.

In Figure-4, there is the invention of intramedullary nail that is inserted in to a canullated fractured bone. The fixation guide wire (37), guides the cannulated fixation screw (37) by getting out of the fourth fixation input (15).

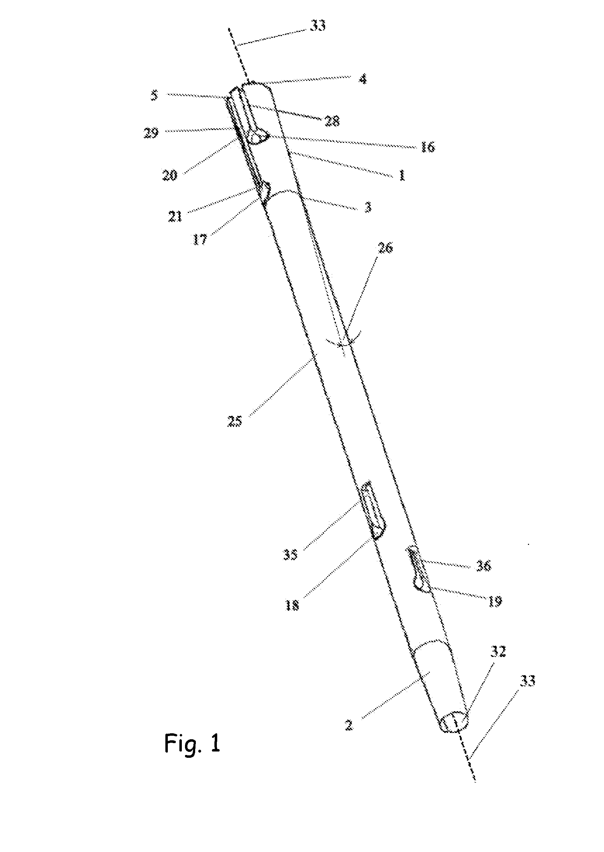

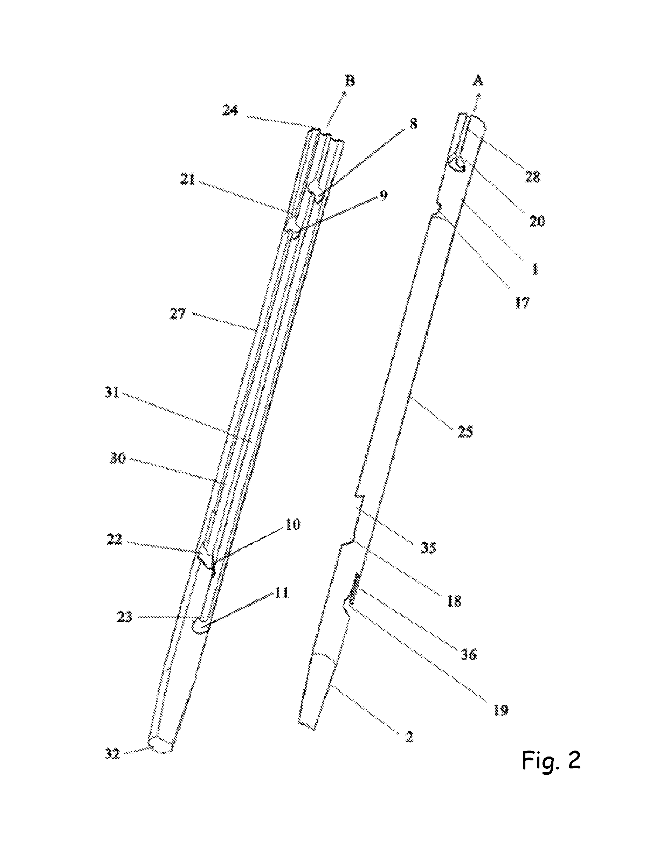

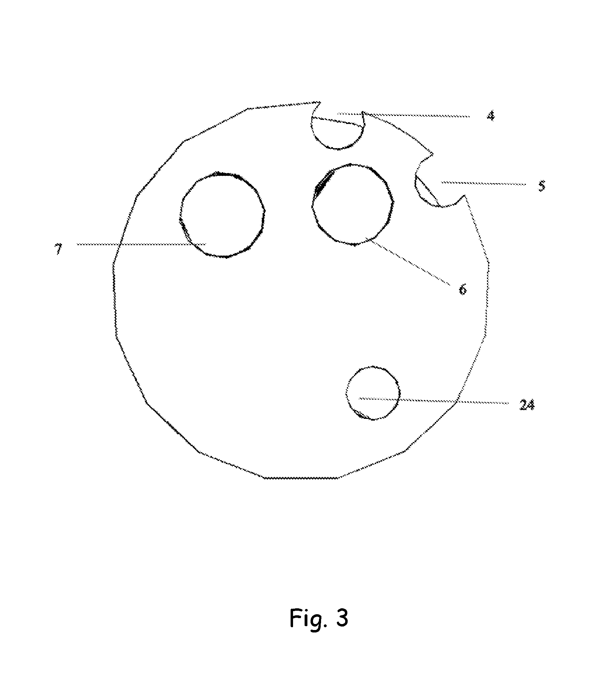

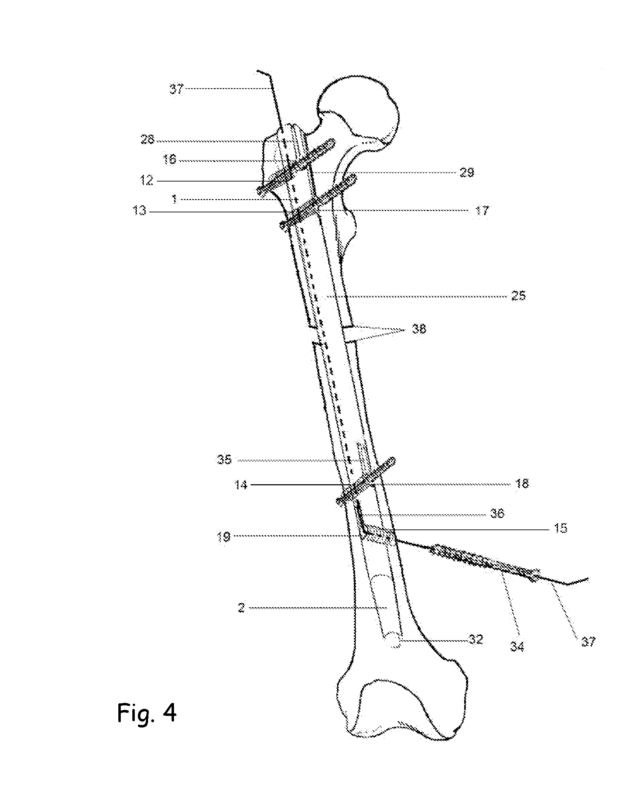

## Claims

1. The invention is multiple cannulated, self guided intramedullary nail that can be used without the need for extra guide systems attached to the intramedullary nail and can be utilized with devices used to detect the fructures in bones, fixing fructures, extremity lengthening, correcting deformities with the multiple grooves and channels in its structure, wherein, it comprises;
• At the proximal end (1) of the intramedullary nail, first groove input (4), first groove (28), first groove angled surfce (20), first groove output (8), first fixation screw input,(12), first fixation screw output (16) which are generating guide ways necessary for the fixation guide wire (37) applied at the proximal end (1) to drill the bone at an appropriate point.
• At the proximal end (1) of the intramedullary nail, second groove input (5), second groove (29), second groove angled surfce (21), second groove output (9), second fixation screw input(13), second fixation screw output (17) which are generating guide ways necessary for the fixation guide wire (37) applied at the proximal end (1) to drill the bone at an appropriate point.
• At the proximal end (1) of the intramedullary nail, first channel input (6), first channel (30) along the nail body (25) , third groove (35) at the distal end (2) of the intramedullary nail, first channel angled surfce (22), third fixation screw input (14), third fixation screw output (18) which are generating guide ways necessary for the fixation guide wire (37) applied at the proximal end (1) to drill the bone at an appropriate point.
• At the proximal end (1) of the intramedullary nail, first channel input (6), first channel (30) along the nail body (25) , third groove (35) at the distal end (2) of the intramedullary nail, first channel angled surfce (22), third fixation screw input,(14), third fixation screw output (18) which are generating guide ways necessary for the fixation guide wire (37) applied at the proximal end (1) to drill the bone at an appropriate point.
• At the proximal end (1) of the intramedullary nail, second channel input (7), second channel (31) along the nail body (25) , fourth groove (36) at the distal end (2) of the intramedullary nail, second channel angled surfce (23), fourth fixation screw input,(15), fourth fixation screw output (19) which are generating guide ways necessary for the fixation guide wire (37) applied at the proximal end (1) to drill the bone at an appropriate point.

2. A multiple cannulated, self guided intramedullary nail according to Claim 1, wherein, it comprises;
- guide output (32), nail guide channel (27) along the nail body (25) at the distal end (2), nail guide input (24) at the proximal end (1) which are determining way of nail guide wire (33) to guide the intramedullary nail to be inserted in to the bone through the bone cavity (38).

3. A multiple cannulated, self guided intramedullary nail accoding to the claims mentioned above; wherein, it comprises;
- A nail neck angle (26) at nail neck (3) area with varying degrees according to the bone feature; providing the compatibility of the intramedullary nail's shape and form for the anatomic structure of the bone which it will be placed in.

4. A multiple cannulated, self guided intramedullary nail accoding to the claims mentioned above; wherein, it comprises;
- That fixation guide wire (37) guides the cannulated fixation screw (34) which fixes the intramedullary nail in to the bone, without integreted an extra guide component in controlled manner.

5. A multiple cannulated, self guided intramedullary nail accoding to the claims mentioned above; wherein, it comprises;
- An appropriate form and shape providing the grooves, groove angled surfaces, channels, fixation screw inputs and fixation screw outputs to orient the fixation guide wire (37) through out the nail in required angle and to drill the bone in required angle.

6. A multiple cannulated, self guided intramedullary nail accoding to the claims mentioned above; wherein, it comprises;
- Providing the fixation guide wire (37) to drill the bone in desired angle and diameter.

7. A multiple cannulated, self guided intramedullary nail accoding to the claims mentioned above; wherein, it comprises;
- Providing the cannulated fixation screw (34) passed through the fixation guide wire (37) to enter to the fixation screw input, exit from the fixation screw output, drill the bone's opposite cortex at the exit side.
